# EUROPEAN PATENT APPLICATION

(11) **EP 4 741 826 A1**
(43) Date of publication of application: **13.05.2026**
(21) Application number: 25842876.2
(22) Date of filing: 10.10.2025
(51) Int. Cl.: G01N 33/543, G01N 33/531, G01N 33/564, G01N 21/76

(54) **ANTI-NEPHRIN AUTOANTIBODY ASSAY KIT, DETECTION METHOD THEREFOR AND PREPARATION METHOD THEREFOR**

(30) Priority: 18.10.2024 CN 202411462363
(71) Applicant: Shenzhen Yhlo Biotech Co., Ltd., Shenzhen, Guangdong 518116 (CN)
(72) Inventor: The designation of the inventor has not yet been filed
(74) Representative: Michalski Hüttermann & Partner Patentanwälte mbB
(86) International application number: PCT/CN2025/126718
(87) International publication number: WO 2026/061548

(57) **Abstract**

The present application relates to a magnetic microparticle specifically binding to an anti-Nephrin autoantibody in a sample, including a magnetic bead coated with a polypeptide or a fragment thereof capable of specifically binding to the anti-Nephrin autoantibody in a mammal; wherein the magnetic bead is a carboxyl magnetic bead, and the particle size of the carboxyl magnetic bead is 1 µm to 5 µm.

## Description

### RELATED APPLICATIONS

The present application claims priority to Chinese Patent Application No. 202411462363.7, filed on October 18, 2024, titled "ANTI-NEPHRIN AUTOANTIBODY ASSAY KITSAND DETECTION METHOD AND PREPARATION METHODS", the entire content of which is incorporated herein by reference.

### TECHNICAL FIELD

The present application relates to the field of in vitro detection technology, and particularly to an anti-Nephrin autoantibody assay kit and detection method and preparation method thereof.

### BACKGROUND

Nephrin protein is mainly present in the slit diaphragm of podocytes in the renal glomerulus and is a membrane protein crucial for maintaining the normal function of the renal glomerular filtration barrier. As early as before 2021, we first discovered and screened podocyte autoantibodies specific for idiopathic nephrotic syndrome and identified the existence of anti-Nephrin autoantibodies¹. However, the concentration of anti-Nephrin autoantibodies in serum is relatively low compared to other podocyte autoantibodies we discovered. Therefore, detections based on conventional enzyme-linked immunosorbent assay (ELISA) and immunoblotting methods result in a low detection rate due to limited sensitivity. Subsequent research found that post-transplant recurrence in patients with focal segmental glomerulosclerosis type nephrotic syndrome is related to Nephrin autoantibodies, suggesting that anti-Nephrin autoantibodies may be an important pathogenic factor^{2,3}. Recent clinical cohort studies further revealed that anti-Nephrin autoantibodies are widely present in patients with minimal change type and idiopathic nephrotic syndrome, closely correlated with disease activity, and serve as effective biomarkers for monitoring disease changes and treatment response^{4,5}. In summary, accumulating evidences indicate that anti-Nephrin autoantibodies hold significant clinical value. There is an urgent clinical need to establish and develop anti-Nephrin antibody detection methods and kits that have short detection time, high sensitivity, strong specificity, stable and reliable results, high uniformity, and ease of clinical promotion and application.

Existing methods for detecting anti-Nephrin antibodies include enzyme-linked immunosorbent assay, radioimmunoassay, etc.. Enzyme-linked immunosorbent assay has insufficient sensitivity, involves lengthy experimental procedures, and reagents show significant batch-to-batch variations. Although radioimmunoassay has high sensitivity and specificity, the use of radioactive tracers leads to radioactive contamination and hazards. Furthermore, commonly used radionuclides have short half-lives, resulting in a limited shelf life for kits, and fail to perform automated analysis, causing significant challenges for clinical promotion and application. There is an urgent need to develop conventional, reliable, efficient, and clinically accessible quantitative detection methods and assay kits for anti-Nephrin autoantibodies.
[1]Ye Q, Zhang Y, Zhuang J, Bi Y, Xu H, Shen Q, Liu J, Fu H, Wang J, Feng C, Tang X, Liu F, Gu W, Zhao F, Zhang J, Qin Y, Shang S, Shen H, Chen X, Shen H, Liu A, Xia Y, Lu Z, Shu Q, Mao J. The important roles and molecular mechanisms of annexin A2 autoantibody in children with nephrotic syndrome. Ann Transl Med. 2021 Sep;9(18):1452.
[2] Hattori M. Anti-nephrin autoantibodies: novel predictors of post-transplant recurrence of focal segmental glomerular sclerosis. Kidney Int. 2024 Oct;106(4):570-572.
[3] Hattori M, Shirai Y, Kanda S, et al. Circulating nephrin autoantibodies and posttransplant recurrence of primary focal segmental glomerulosclerosis. Am J Transplant. 2022 Oct;22(10):2478-2480.
[4] Watts AJB, Keller KH, Lerner G, et al. Discovery of Autoantibodies Targeting Nephrin in Minimal Change Disease Supports a Novel Autoimmune Etiology. J Am Soc Nephrol. 2022 Jan;33(1):238-252. doi: 10.1681/ASN.2021060794.
[5] Tomas NM, Hengel FE, Huber TB. Autoantibodies Targeting Nephrin in Podocytopathies. Reply. N Engl J Med. 2024 Oct 10;391(14):1367-1368. doi: 10.1056/NEJMc2410840.

### SUMMARY

To address the technical problems in the prior art, a first objective of the present application is to provide an anti-Nephrin autoantibody assay kit, which can solve the problems of low accuracy, low sensitivity, and low signal-to-noise ratio of existing kits.

A second objective of the present application is to provide a detection method for the anti-Nephrin autoantibody assay kit, which can solve the problems of complex procedures and long detection time associated with detection methods of existing kits.

A third objective of the present application is to provide a preparation method for the anti-Nephrin autoantibody assay kit, which can solve the problems of low process stability, difficult operation, high production costs, and difficulty in clinical promotion of existing kits.

The first and third objectives of the present application are achieved by the following technical solutions:

A magnetic microparticle specifically binding to an anti-Nephrin antibody in a sample, includes a Nephrin antigen and a magnetic bead, and the Nephrin antigen being coated on the surface of the magnetic bead; wherein the magnetic bead is a carboxyl magnetic bead; a particle size of the carboxyl magnetic bead is 1 µm to 5 µm; optionally, the particle size of the carboxyl magnetic bead is 1 µm to 3 µm; and optionally, a particle size of the magnetic bead is 3 µm.

According to the above magnetic microparticle, the Nephrin antigen is a polypeptide or fragment thereof capable of specifically binding to an anti-Nephrin autoantibody in a mammal, and a sequence of the Nephrin antigen is as set forth in SEQ ID NO: 1.

According to the above magnetic microparticle, the magnetic microparticle is blocked by a blocking agent. Optionally, the blocking agent includes 1% (w/v) of bovine serum albumin, 0.5% (w/v) of casein, or 0.5% (w/v) of fish gelatin.

A preparation method for a magnetic microparticle specifically binding to an anti-Nephrin antibody in a sample, includes: obtaining a mixed solution including an activated magnetic bead; adding a Nephrin antigen to the mixed solution, and mixing uniformly, wherein the Nephrin antigen is a polypeptide or fragment thereof capable of specifically binding to an anti-Nephrin autoantibody in a mammal; suspending and reacting the mixed solution for 1 to 2 hours at a temperature in a range of 20 to 25 °C; washing the reacted product with a blocking agent, wherein the blocking agent includes 1% (w/v) of bovine serum albumin, 0.5% (w/v) of casein, or 0.5% (w/v) of fish gelatin; and obtaining a Nephrin antigen-coated magnetic bead.

The above method further includes diluting the Nephrin antigen-coated magnetic bead in a diluent to obtain a magnetic microparticle-diluted solution; wherein a concentration of the magnetic microparticle in the magnetic microparticle-diluted solution is 0.1 to 0.2 mg/mL.

According to the above method, the diluent includes (0.80 to 1.30) g/L of tris(hydroxymethyl)aminomethane, (5.00 to 8.00) g/L of tris(hydroxymethyl)aminomethane hydrochloride, (7.00 to 11.00) g/L of sodium chloride, (1.00 to 5.00) g/L of Triton-X 100, (0.20 to 0.70) g/L of Tween-20, and (0.20 to 0.70) g/L of ProClin 300; optionally, the diluent includes 1.12 g/L of tris(hydroxymethyl)aminomethane, 6.42 g/L of tris(hydroxymethyl)aminomethane hydrochloride, 9.00 g/L of sodium chloride, 3.00 g/L of Triton-X 100, 0.50 g/L of Tween-20, and 0.50 g/L of ProClin 300.

According to the above method, the blocking agent includes fish gelatin.

According to the above method, a ratio of the magnetic bead added to the Nephrin antigen is: 5 to 20 µg of the Nephrin antigen added to a magnetic bead diluent containing about 1 mg of the magnetic bead; and optionally, 10 to 20 µg of the Nephrin antigen is added.

According to the above method, the magnetic bead is carboxyl magnetic bead.

According to the above method, the particle size of the magnetic bead is 1 µm to 5 µm; optionally, the particle size of the magnetic bead is 1 µm to 3 µm; and more optionally, the particle size of the magnetic bead is 3 µm.

An anti-Nephrin autoantibody assay kit includes the magnetic microparticle in any embodiment described above, or the magnetic micropaticle prepared by any one of the above preparation methods.

The above kit further includes a chemiluminescent label-labeled IgG antibody; and optionally, a concentration of the chemiluminescent label-labeled IgG antibody is 0.02 to 0.04 µg/mL.

According to the above kit, the chemiluminescent label is selected from acridinium ester, acridinium sulfonamide, acridinium toluenesulfonamide, acridinium p-methylsulfonamide, or acridinium trifluoromethanesulfonamide; and optionally, the chemiluminescent label is acridinium ester.

According to the above kit, the chemiluminescent label-labeled IgG antibody is diluted in a label diluent, wherein the label diluent includes (0.30 to 0.70) g/L of sodium dihydrogen phosphate, (15.00 to 18.00) g/L of disodium hydrogen phosphate, (35.00 to 45.00) g/L of sodium chloride, (7.00 to 13.00) g/L of bovine serum albumin, (3.00 to 7.00) g/L of Triton-X 405, and (0.30 to 0.70) g/L of ProClin 300; and optionally, the label diluent includes 0.50 g/L of sodium dihydrogen phosphate, 16.75 g/L of disodium hydrogen phosphate, 40.00 g/L of sodium chloride, 10.00 g/L of bovine serum albumin, 5.00 g/L of Triton-X 405, and 0.50 g/L of ProClin 300.

The above kit further includes a sample diluent. The sample diluent includes (0.50 to 1.50) g/L of tris(hydroxymethyl)aminomethane, (5.00 to 8.00) g/L of tris(hydroxymethyl)aminomethane hydrochloride, (7.00 to 11.00) g/L of sodium chloride, and (17.00 to 23.00) g/L of bovine serum albumin.

Optionally, the sample diluent includes 1.00 g/L of tris(hydroxymethyl)aminomethane, 6.58 g/L of tris(hydroxymethyl)aminomethane hydrochloride, 9.00 g/L of sodium chloride, and 20.00 g/L of bovine serum albumin.

Optionally, the sample diluent further includes (0.30 to 0.70) g/L of Tween 20, and (0.30 to 0.70) g/L of ProClin 300. Optionally, the sample diluent further includes 0.50 g/L of Tween 20, and 0.50 g/L of ProClin 300.

The above kit further includes a calibrator, the calibrator including an anti-Nephrin autoantibody, and a calibrator buffer solution. Optionally, the calibrator buffer solution includes (0.10 to 0.30) g/L of tris(hydroxymethyl)aminomethane, (1.00 to 1.60) g/L of tris(hydroxymethyl)aminomethane hydrochloride, (7.00 to 11.00) g/L of sodium chloride, (3.00 to 7.00) g/L of bovine serum albumin, (0.30 to 0.70) g/L of Tween, and (0.30 to 0.70) g/L of ProClin 300. Further optionally, the calibrator buffer solution includes 0.20 g/L of tris(hydroxymethyl)aminomethane, 1.32 g/L of tris(hydroxymethyl)aminomethane hydrochloride, 9.00 g/L of sodium chloride, 5.00 g/L of bovine serum albumin, 0.50 g/L of Tween, and 0.50 g/L of ProClin 300.

The second objective of the present application is achieved by the following technical solution:

A method for detecting an anti-Nephrin autoantibody in a sample using the Nephrin antigen-coated magnetic bead prepared by any one of the above methods, the above Nephrin antigen-coated magnetic bead, or any one of the above kits, includes: adding a sample to be tested to a sample diluent and mixing uniformly; adding a Nephrin antigen-coated magnetic bead or a magnetic microparticle-diluted solution, and reacting for a certain time; adding acridinium ester-labeled mouse anti-human IgG antibody or acridinium ester-labeled mouse anti-human IgG antibody diluted in a label diluent, and reacting for a certain time; adding a pre-excitation solution and an excitation solution; and detecting a relative luminescence intensity.

According to the above method, the pre-excitation solution includes (0.80 to 1.70)% (w/v) of hydrogen peroxide, and the excitation solution includes (0.2 to 0.5) mol/L of sodium hydroxide. Optionally, the pre-excitation solution includes 1.32% (w/v) of hydrogen peroxide, and the excitation solution includes 0.35 mol/L of sodium hydroxide.

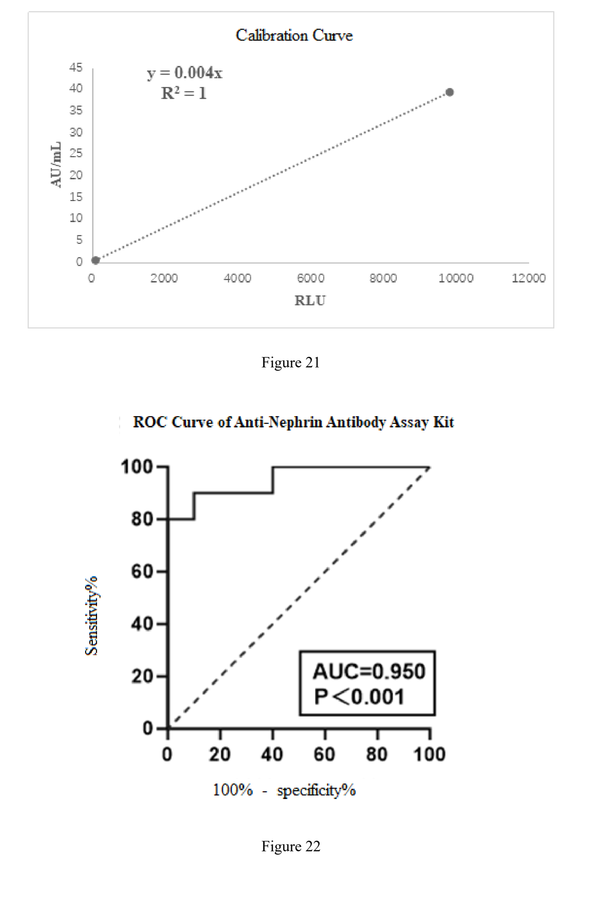

The above method further includes: obtaining concentrations of a calibrator and luminescence intensity values thereof, and determining a calibration curve, as shown in Figure 21, with the concentration of the calibrator of 0.42 AU/mL for CAL1 and 39.3 AU/mL for CAL2; matching the relative luminescence intensity of the sample with the calibration curve of luminescence intensity values of the calibrator to obtain a content of the anti-Nephrin autoantibody in the sample to be tested.

According to the above method, a volume ratio of the sample to be tested, the magnetic microparticle-diluted solution, the acridinium ester-labeled mouse anti-human IgG antibody diluted in the label diluent, and the sample diluent that are added is (15 to 25): (40 to 60): (80 to 120): (80 to 120), preferably 20: 50: 100: 100.

According to the above method, the reaction after adding the Nephrin antigen-coated magnetic bead or the magnetic microparticle-diluted solution is performed for 10 to 20 min.

According to the above method, the sample is serum.

Details of one or more embodiments of the present application are set forth in the following description. Other features, objectives, and advantages of the present application will become apparent from the description and claims.

### BRIEF DESCRIPTION OF THE DRAWINGS

The preferred embodiments of the present application will be further described in detail below with reference to the accompanying drawings, in which:
Figures 1 to 10 show indirect immunofluorescence images of positive samples according to an embodiment of the present application.
Figures 2 to 20 show indirect immunofluorescence images of negative samples according to an embodiment of the present application.
Figure 21 shows a calibration curve of an anti-Nephrin autoantibody kit according to an embodiment of the present application.
Figure 22 shows an ROC curve of detection results of the anti-Nephrin autoantibody kit for samples according to the embodiment.

### DETAILED DESCRIPTION

To make the objectives, technical solutions, and advantages of the embodiments of the present application more clear the technical solutions in the embodiments of the present application will be described clearly and completely below with reference to the accompanying drawings in the embodiments of the present application. It is obvious that the described embodiments are only some of the embodiments of the present application, and not all of them. Based on the embodiments in the present application, all other embodiments obtained by a person of ordinary skill in the art without creative effort shall fall within the protection scope of the present application.

In the present application, the "anti-Nephrin autoantibody" is a key protein in the complex podocyte slit diaphragm structure, and has broad signaling functions. Severe podocyte injury occurs when there is gene mutation or experimental Nephrin knockout. The anti-Nephrin autoantibody is widely present in patients with nephrotic syndrome across all age groups, and induces nephrotic syndrome, Nephrin phosphorylation, and cellular rearrangement at the proteomic level, leading to the typical ultrastructural changes in minimal change nephropathy.

In the present application, the "Nephrin antigen" refers to any polypeptide or fragment thereof capable of specifically binding to an anti-Nephrin autoantibody in a mammal, including humans. In some embodiments, the Nephrin antigen refers to a polypeptide of the extracellular segment of human recombinant Nephrin protein. Further, in some embodiments, the sequence of the Nephrin antigen is as shown in SEQ ID NO: 1 or SEQ ID NO: 2.
SEQ ID NO: 1 
MGWSCIILFLVATATGVHSQLAIPASVPRGFWALPENLTVVEGASVELRCGVSTPGSAVQW
AKDGLLLGPDPRIPGFPRYRLEGDPARGEFHLHIEACDLSDDAEYECQVGRSEMGPELVSPRVILSILVP
PKLLLLTPEAGTMVTWVAGQEYVVNCVSGDAKPAPDITILLSGQTISDISANVNEGSQQKLFTVEATAR
VTPRSSDNRQLLVCEASSPALEAPIKASFTVNVLFPPGPPVIEWPGLDEGHVRAGQSLELPCVARGGNP
LATLQWLKNGQPVSTAWGTEHTQAVARSVLVMTVRPEDHGAQLSCEAHNSVSAGTQEHGITLQVTFP
PSAIIILGSASQTENKNVTLSCVSKSSRPRVLLRWWLGWRQLLPMEETVMDGLHGGHISMSNLTFLAR
REDNGLTLTCEAFSEAFTKETFKKSLILNVKYPAQKLWIEGPPEGQKLRAGTRVRLVCLAIGGNPEPSL
MWYKDSRTVTESRLPQESRRVHLGSVEKSGSTFSRELVLVTGPSDNQAKFTCKAGQLSASTQLAVQFP
PTNVTILANASALRPGDALNLTCVSVSSNPPVNLSWDKEGERLEGVAAPPRRAPFKGSAAARSVLLQV
SSRDHGQRVTCRAHSAELRETVSSFYRLNVLYRPEFLGEQVLVVTAVEQGEALLPVSVSANPAPEAFN
WTFRGYRLSPAGGPRHRILSSGALHLWNVTRADDGLYQLHCQNSEGTAEARLRLDVHYAPTIRALQD
PTEVNVGGSVDIVCTVDANPILPGMFNWERLGEDEEDQSLDDMEKISRGPTGRLRIHHAKLAQAGAY
QCIVDNGVAPPARRLLRLVVRFAPQVEHPTPLTKVAAAGDSTSSATLHCRARGVPNIVFTWTKNGVPL
 
DLQDPRYTEHTYHQGGVHSSLLTIANVSAAQDYALFTCTATNALGSDQTNIQLVSISRPDPPSGLKVVS
LTPHSVGLEWKPGFDGGLPQRFCIRYEALGTPGFHYVDVVPPQATTFTLTGLQPSTRYRVWLLASNAL
GDSGLADKGTQLPITTPGLHQPSGEPEDQLPTEPPSGGS.
SEQ ID NO: 2

MGWSCIILFLVATATGVHSQLAIPASVPRGFWALPENLTVVEGASVELRCGVSTPGSAVQW
AKDGLLLGPDPRIPGFPRYRLEGDPARGEFHLHIEACDLSDDAEYECQVGRSEMGPELVSPRVILSILVP
PKLLLLTPEAGTMVTWVAGQEYVVNCVSGDAKPAPDITILLSGQTISDISANVNEGSQQKLFTVEATAR
VTPRSSDNRQLLVCEASSPALEAPIKASFTVNVLFPPGPPVIEWPGLDEGHVRAGQSLELPCVARGGNP
LATLQWLKNGQPVSTAWGTEHTQAVARSVLVMTVRPEDHGAQLSCEAHNSVSAGTQEHGITLQVTFP
PSAIIILGSASQTENKNVTLSCVSKSSRPRVLLRWWLGWRQLLPMEETVMDGLHGGHISMSNLTFLAR
REDNGLTLTCEAFSEAFTKETFKKSLILNVKYPAQKLWIEGPPEGQKLRAGTRVRLVCLAIGGNPEPSL
MWYKDSRTVTESRLPQESRRVHLGSVEKSGSTFSRELVLVTGPSDNQAKFTCKAGQLSASTQLAVQFP
PTNVTILANASALRPGDALNLTCVSVSSNPPVNLSWDKEGERLEGVAAPPRRAPFKGSAAARSVLLQV
SSRDHGQRVTCRAHSAELRETVSSFYRLNVLYRPEFLGEQVLVVTAVEQGEALLPVSVSANPAPEAFN
WTFRGYRLSPAGGPRHRILSSGALHLWNVTRADDGLYQLHCQNSEGTAEARLRLDVHYAPTIRALQD
PTEVNVGGSVDIVCTVDANPILPGMFNWERLGEDEEDQSLDDMEKISRGPTGRLRIHHAKLAQAGAY
QCIVDNGVAPPARRLLRLVVRFAPQVEHPTPLTKVAAAGDSTSSATLHCRARGVPNIVFTWTKNGVPL
DLQDPRYTEHTYHQGGVHSSLLTIANVSAAQDYALFTCTATNALGSDQTNIQLVSISRPDPPSGLKVVS
LTPHSVGLEWKPGFDGGLPQRFCIRYEALGTPGFHYVDVVPPQATTFTLTGLQPSTRYRVWLLASNAL
GDSGLADKGTQLPITTPGLHQPSGEPEDQLPTEPPSGGSGLNDIFEAQKIEWHEGGSHHHHHHHHHH.

In the present application, the "magnetic bead coating amount" refers to the ratio of the extracellular segment of the human recombinant Nephrin protein (Nephrin antigen) added to an activated magnetic bead during preparation of magnetic microparticles. For example, adding 10 µg Nephrin antigen to per mL of the activated magnetic bead can be considered as a coating amount of 10 µg/mL.

In the present application, the "sample" refers to a serum of a subject.

The present application relates to a magnetic microparticle specifically binding to the anti-Nephrin antibody in a sample, the magnetic microparticle including a Nephrin antigen and a magnetic bead, the Nephrin antigen being coated on the surface of the magnetic bead; wherein the magnetic bead is a carboxyl magnetic bead; and the particle size of the carboxyl magnetic bead is 1 µm to 5 µm; optionally, the particle size of the carboxyl magnetic bead is 1 µm to 3 µm; and optionally, the particle size of the magnetic bead is 3 µm.

In some embodiments, the Nephrin antigen is a polypeptide or a fragment thereof capable of specifically binding to an anti-Nephrin autoantibody in a mammal, and the Nephrin antigen has a sequence as set forth in SEQ ID NO: 1.

In some embodiments, the magnetic microparticle is blocked by a blocking agent. Optionally, the blocking agent includes 1% (w/v) of bovine serum albumin, 0.5% (w/v) of casein, or 0.5% (w/v) of fish gelatin.

The present application relates to a method for preparing a magnetic microparticle specifically binding to an anti-Nephrin autoantibody in a sample, including: obtaining a mixed solution including 1 mg of an activated magnetic bead; adding 5 to 20 µg of a Nephrin antigen to the mixed solution, optionally adding 10 to 20 µg of the Nephrin antigen, and mixing uniformly, wherein the Nephrin antigen is a polypeptide or fragment thereof capable of specifically binding to an anti-Nephrin autoantibody in a mammal; suspending and reacting the mixed solution for 1 to 2 hours at a temperature of 20 to 25°C; washing the reacted product with a blocking agent, wherein the blocking agent includes 2% (w/v) of bovine serum albumin, 2% (w/v) of casein, or 2% (w/v) of fish gelatin, optionally, the blocking agent includes fish gelatin; and obtaining a Nephrin antigen-coated magnetic bead.

In some embodiments, the method for coating the magnetic bead with the Nephrin antigen further includes diluting the Nephrin antigen-coated magnetic bead in a diluent to obtain a magnetic microparticle-diluted solution, wherein the concentration of the magnetic microparticle in the magnetic microparticle-diluted solution is 0.1 to 0.2 mg/mL.

In some embodiments, the diluent includes: (0.80 to 1.30) g/L of tris(hydroxymethyl)aminomethane, (5.00 to 8.00) g/L of tris(hydroxymethyl)aminomethane hydrochloride, (7.00 to 11.00) g/L of sodium chloride, (1.00 to 5.00) g/L of Triton-X 100, (0.20 to 0.70) g/L of Tween-20, and (0.20 to 0.70) g/L of ProClin 300. Optionally, the diluent includes 1.12 g/L of tris(hydroxymethyl)aminomethane, 6.42 g/L of tris(hydroxymethyl)aminomethane hydrochloride, 9.00 g/L of sodium chloride, 3.00 g/L of Triton-X 100, 0.50 g/L of Tween-20, and 0.50 g/L of ProClin 300.

In some embodiments, the magnetic bead is a carboxyl magnetic bead. In some embodiments, the particle size of the magnetic bead is 1 µm to 5 µm; optionally, the particle size of the magnetic bead is 1 µm to 3 µm; more optionally, the particle size of the magnetic bead is 3 µm.

The present application relates to an anti-Nephrin autoantibody assay kit, including the magnetic microparticle in any embodiment described above, or the magnetic microparticle prepared by any one of the preparation methods.

In some embodiments, the kit further includes a chemiluminescent label-labeled IgG antibody; optionally, a concentration of the chemiluminescent label-labeled IgG antibody is 0.02 to 0.04 µg/mL.

In some embodiments, the chemiluminescent label is selected from acridinium ester, acridinium sulfonamide, acridinium toluenesulfonamide, acridinium p-methylsulfonamide, or acridinium trifluoromethanesulfonamide. Optionally, the chemiluminescent label is acridinium ester.

In some embodiments, the chemiluminescent label-labeled IgG antibody is diluted in a label diluent. The label diluent includes (0.30 to 0.70) g/L of sodium dihydrogen phosphate; (15.00 to 18.00) g/L of disodium hydrogen phosphate, (35.00 to 45.00) g/L of sodium chloride, (7.00 to 13.00) g/L of bovine serum albumin, (3.00 to 7.00) g/L of Triton-X 405, and (0.30 to 0.70) g/L of ProClin 300. Optionally, the label diluent includes 0.50 g/L of sodium dihydrogen phosphate, 16.75 g/L of disodium hydrogen phosphate, 40.00 g/L of sodium chloride, 10.00 g/L of bovine serum albumin, 5.00 g/L of Triton-X 405, and 0.50 g/L of ProClin 300.

In some embodiments, the kit further includes a sample diluent. The sample diluent includes (0.50 to 1.50) g/L of tris(hydroxymethyl)aminomethane, (5.00 to 8.00) g/L of tris(hydroxymethyl)aminomethane hydrochloride, (7.00 to 11.00) g/L of sodium chloride, and (17.00 to 23.00) g/L of bovine serum albumin. Optionally, the sample diluent includes 1.00 g/L of tris(hydroxymethyl)aminomethane, 6.58 g/L of tris(hydroxymethyl)aminomethane hydrochloride, 9.00 g/L of sodium chloride, and 20.00 g/L of bovine serum albumin.

Optionally, the sample diluent further includes (0.30 to 0.70) g/L of Tween 20, and (0.30 to 0.70) g/L of ProClin 300. Optionally, the sample diluent further includes 0.50 g/L of Tween 20, and 0.50 g/L of ProClin 300.

In some embodiments, the kit further includes a calibrator, the calibrator including an anti-Nephrin autoantibody, and a calibrator buffer solution. Optionally, the calibrator buffer solution includes (0.10 to 0.30) g/L of tris(hydroxymethyl)aminomethane, (1.00 to 1.60) g/L of tris(hydroxymethyl)aminomethane hydrochloride, (7.00 to 11.00) g/L of sodium chloride, (3.00 to 7.00) g/L of bovine serum albumin, (0.30 to 0.70) g/L of Tween, and (0.30 to 0.70) g/L of ProClin 300. Further optionally, the calibrator buffer solution includes 0.20 g/L of tris(hydroxymethyl)aminomethane, 1.32 g/L of tris(hydroxymethyl)aminomethane hydrochloride, 9.00 g/L of sodium chloride, 5.00 g/L of bovine serum albumin, 0.50 g/L of Tween, and 0.50 g/L of ProClin 300.

In some embodiments, the calibrator includes an anti-Nephrin autoantibody. In some embodiments, the calibrator includes a human anti-Nephrin autoantibody. In some embodiments, the anti-Nephrin autoantibody in the calibrator is obtained from a clinical anti-Nephrin autoantibody-positive patient. In some embodiments, the anti-Nephrin autoantibody in a sample from a subject is detected, and the anti-Nephrin autoantibody in samples from clinical anti-Nephrin autoantibody-positive patients are obtained.

The present application relates to a method for detecting an anti-Nephrin autoantibody in a sample using the Nephrin antigen-coated magnetic bead prepared by any one of the above methods, the above Nephrin antigen-coated magnetic bead, or any one of the above kits, including adding a sample to be tested to a sample diluent and mixing uniformly; adding a Nephrin antigen-coated magnetic bead or a magnetic microparticle-diluted solution, and reacting for a certain time; adding an acridinium ester-labeled mouse anti-human IgG antibody or an acridinium ester-labeled mouse anti-human IgG antibody diluted in a label diluent, and reacting for a certain time; adding a pre-excitation solution and an excitation solution; and detecting a relative luminescence intensity.

In some embodiments, the pre-excitation solution includes (0.80 to 1.70)% (w/v) of hydrogen peroxide, and the excitation solution includes (0.2 to 0.5) mol/L of sodium hydroxide. Optionally, the pre-excitation solution includes 1.32% (w/v) of hydrogen peroxide, and the excitation solution includes 0.35 mol/L of sodium hydroxide.

In some embodiments, the method for detecting an anti-Nephrin autoantibody in a sample further includes: obtaining concentrations of a calibrator and luminescence intensity values thereof, and determining a calibration curve, as shown in Figure 22, with the concentration of the calibrator of 0.42 AU/mL for CAL1 and 39.3 AU/mL for CAL2; and matching the relative luminescence intensity of the sample with the calibration curve of luminescence intensity values of the calibrator to obtain a content of the anti-Nephrin autoantibody in the sample to be tested.

In some embodiments, a volume ratio of the sample to be tested, the magnetic microparticle-diluted solution, the acridinium ester-labeled mouse anti-human IgG antibody diluted in the label diluent, and the sample diluent that are added is (15 to 25): (40 to 60): (80 to 120): (80 to 120), preferably 20: 50: 100: 100.

In some embodiments, the reaction after adding the Nephrin antigen-coated magnetic bead or the magnetic microparticle-diluted solution is performed for 10 to 20 min.

The preferred embodiments of the present application will be further described in detail below with reference to the accompanying drawings. The detection standard for the embodiments of the present application refers to In vitro diagnostic test systems-Performance evaluation method according to YY/T 1789.4-2022.

In the following embodiments, samples obtained from subjects clinically confirmed as positive for the anti-Nephrin autoantibody were selected as the experimental group, and samples obtained from subjects clinically confirmed as negative for the anti-Nephrin autoantibody were selected as the control group. Both the experimental and control groups included children aged from 3 to 18 years, all suffering from nephrotic syndrome at varying degrees.

Further, the present application relates to a method for measuring an anti-Nephrin autoantibody in an *ex vivo* sample from a subject. In this method, the magnetic bead coated with a polypeptide or fragment thereof that specifically binds to the anti-Nephrin autoantibody (hereinafter referred to as the magnetic microparticle), and a complex of a chemiluminescent label-labeled IgG antibody (hereinafter referred to as a label antigen) are utilized. Upon the magnetic microparticle and the label antigen undergo an immune reaction to form a magnetic microparticle-label antigen complex, the acridinium ester decomposes and emits light in an alkaline environment formed by the oxidant hydrogen peroxide and the sodium hydroxide, thereby determining the detection result for the anti-Nephrin autoantibody.

Compared with conventional techniques, the beneficial effects of the present application lie in that:
According to the ratio of the Nephrin antigen-coated magnetic microparticle working solution as a R1 component to the acridinium ester-labeled mouse anti-human IgG working solution as a R2 component in the kit of the present application, the magnetic bead can bind more antigens at the same proportion, thereby improving the capture effect for antibody in the sample to be tested, significantly enhancing the signal-to-noise ratio and sensitivity for the detection, and enabling quantitative detection of the concentration of the anti-Nephrin autoantibody in the sample.

The detection method for the kit of the present application, through selective capture of target molecules by the magnetic beads, shows a simple and fast operational workflow, is easy to automate and promotes clinical application, and yields highly uniform and accurate results within 30 minutes, greatly improving sample processing efficiency. Meanwhile, the detection method performs the serial dilution, which reduces usage costs and expands the reportable range.

The preparation method for the kit of the present application has a simple process flow, and the prepared products exhibit good sensitivity, specificity, and stability.

### Example 1: Preparation Method for the Anti-Nephrin Autoantibody Assay Kit

### 1. Preparation method for R1 reagent - magnetic microparticles

In this example, the magnetic beads can be Toysl magnetic beads or carboxyl magnetic beads of different particle sizes. Adjustments to particle size and coating amount of the magnetic bead refer to Example 3 and are not repeated herein. In this example, the Toysl magnetic beads used were purchased from JSR Corporation, with a product batch number MS300/Tosyl; the carboxyl magnetic beads used were purchased from JSR Corporation, with a product batch number MS300/Carboxyl. The polypeptide or fragment thereof (antigen) capable of specifically binding to the anti-Nephrin autoantibody involved in this example is the extracellular segment of human recombinant Nephrin protein.

Furthermore, during screening and optimization of magnetic beads in Example 3, only the corresponding parameters were replaced based on this example, while other parameters remained unchanged.

In this example, the preparation method for magnetic microparticles includes:
Magnetic beads were added into 1-Ethyl-3-(3-dimethylaminopropyl)carbodiimide (EDC) and N-Hydroxysulfosuccinimide sodium salt (Sulfo-NHS), which are chemical crosslinking agents, to activate functional groups on their surfaces.

1 mg of activated magnetic beads were coated and bound with 5 µg, 10 µg, 15 µg, or 20 µg of human recombinant Nephrin protein extracellular segment (i.e., a Nephrin antigen), so that the coating amounts of the magnetic beads were 5 µg/mg, 10 µg/mg, 15 µg/mg, or 20 µg/mg, respectively. The coating and binding reaction was conducted by suspending the mixture for reacting for 1 to 2 hours in an environment at 20 to 25°C.

The coated magnetic beads were then washed with a blocking agent to obtain Nephrin antigen-coated magnetic beads; and components of the blocking agent can refer to Example 3.

Subsequently, a diluent (including, per 1 L: 950.00 g ultrapure water, 1.12 g tris(hydroxymethyl)aminomethane, 6.42 g tris(hydroxymethyl)aminomethane hydrochloride, 9.00 g sodium chloride, 3.00 g Triton-X 100, 0.50 g Tween-20, and 0.50 g ProClin 300 was added for dilution to obtain a Nephrin antigen-coated magnetic bead working solution. According to an example of the present application, the concentration of the magnetic microparticle working solution was 0.1 mg/mL.

### 2. Preparation method for R2 reagent - labeled antibodies

In this example, a chemiluminescent label was used to label the IgG antibody. The IgG antibody used herein was mouse anti-human IgG antibody, purchased from GenScript Corporation, with a product batch number A01855. In the present application, the chemiluminescent label is not limited; for example, acridinium ester, acridinium sulfonamide, acridinium toluenesulfonamide, acridinium p-methylsulfonamide, acridinium trifluoromethanesulfonamide, etc., can all be used to label the IgG antibody. Further, in this example, the chemiluminescent label for labeling the IgG antibody is acridinium ester, purchased from Seebio, with a product batch number EKY0980A.

In this example, the method for labeling the mouse anti-human IgG antibody with acridinium ester was as follows.

The mouse anti-human IgG antibody was incubated and bound with acridinium ester, in which a ratio of acridinium ester bound to the mouse anti-human IgG antibody was 8 µg acridinium ester per 100 µg antibody.

The binding was conducted by reacting acridinium ester and the mouse anti-human IgG antibody in the above ratio at room temperature in the dark for 2 hours, then adding a reaction termination solution including 0.03 mol lysine. Next, the reaction continued at room temperature in the dark for 1 hour. After completion, a desalting column was used to purify the acridinium ester labeled antibody.

After purification, an acridinium ester diluent (including, per 1 L: 965.00 g ultrapure water, 0.50 g sodium dihydrogen phosphate, 16.75 g disodium hydrogen phosphate, 40.00 g sodium chloride, 10.00 g bovine serum albumin, 5.00 g Triton-X 405, and 0.50 g ProClin 300) was added for dilution to obtain an acridinium ester-labeled mouse anti-human IgG working solution. In some examples, the concentration of the labeled antibody working solution was 0.02 µg/mL.

### 3. Preparation method for R3 reagent - a sample diluent

In this example, the sample diluent included per 1 L, 976.00 g ultrapure water, 1.00 g tris(hydroxymethyl)aminomethane, 6.58 g tris(hydroxymethyl)aminomethane hydrochloride, 9.00 g sodium chloride, 20.00 g bovine serum albumin, 0.50 g Tween, and 0.50 g ProClin 300. The concentrations of the sample diluent formula were selected according to conventional concentrations.

### 4. Preparation method for R4 reagent - a calibrator buffer solution

In this example, a high-value serum sample was diluted 5-fold with a calibrator buffer solution. The preparation method for the calibrator buffer solution included: per 1 L, adding 990.00 g ultrapure water, 0.20 g tris(hydroxymethyl)aminomethane, 1.32 g tris(hydroxymethyl)aminomethane hydrochloride, 9.00 g sodium chloride, 5.00 g bovine serum albumin, 0.50 g Tween, and 0.50 g ProClin 300. The calibrator buffer solution was stored at 2 to 8°C for later use.

### Example 2: Method for Determining Anti-Nephrin Autoantibodies in Samples

### 1. Chemiluminescence

The sample referred to in this example is serum of a subject.

The method for detecting a sample from a subject using the anti-Nephrin autoantibody assay kit of Example 1 was as follows.

20 µL of the sample to be tested was added to 100 µL of the R3 reagent (i.e., the sample diluent) for dilution.

The diluted sample to be tested was incubated with 50 µL of the R1 reagent (i.e., the magnetic microparticle working solution) at room temperature for 5 to 20 minutes, and a first washing was performed 4 times.

Then, 100 µL of the R2 reagent (i.e., labeled antibody working solution) was added and incubated at room temperature for 10 minutes, and a second washing was performed 4 times.

Next, a pre-excitation solution and an excitation solution were added, with maximum luminescence intensity at 0.4 seconds. The pre-excitation solution included 1.32% (w/v) of hydrogen peroxide, and the excitation solution included 0.35 mol/L of sodium hydroxide.

The relative light unit (RLU) of the final reaction product was detected at a wavelength of 430 nm.

### 3. Indirect Immunofluorescence

Indirect immunofluorescence is the gold standard for detecting antibodies in samples, and the results can be used as a control for results detected by the chemiluminescence of the present application.

Specific experimental steps for indirect immunofluorescence were as follows.

3.1 Culturing and preparing normal human podocytes: normal human podocytes were seeded on a cover slip, and cultured to a monolayer in a suitable medium. The cells were washed three times with PBS, for 5 minutes each time. The normal human podocytes were obtained from an immortalized human podocyte cell line.

3.2 Cell fixation: cells were fixed with 4% paraformaldehyde at room temperature for 10 to 15 minutes. After fixation, the cells were washed three times with PBS, for 5 minutes each time.

3.3 Blocking: nonspecific binding sites were blocked using PBS containing 5% to 10% normal goat serum or BSA by incubation at room temperature for 30 minutes.

3.4 Primary antibody incubation: the cover slip was placed in a serum sample, and incubated at 4°C overnight.

3.5 Washing: After incubation, the cover slip was washed three times with PBS to remove unbound antibodies, for 5 minutes each time.

3.6 Secondary antibody incubation: fluorescently-labeled anti-human IgG secondary antibody was used and incubated with the cover slip at room temperature in the dark for 1 hour.

3.7 Washing: After incubation, the cover slip was washed three times with PBS to remove unbound antibodies, for 5 minutes each time.

3.8 Mounting and observation: the cover slip was mounted with anti-fade mounting medium and observed with a laser scanning confocal microscope, and fluorescent images of cells were captured.

### Example 3: Screening and Optimization of Magnetic Beads

In this example, various parameters for preparing magnetic microparticles were optimized to obtain optimal Nephrin antigen-coated magnetic beads.

### 1. Influence of Different Types of Magnetic Beads on Sample Reactivity

In this example, 10 clinical anti-Nephrin autoantibody-positive samples and 10 clinical anti-Nephrin autoantibody-negative samples were selected. Nephrin antigen was bound to Toysl magnetic beads and carboxyl magnetic beads, respectively. Using other reagents from the kit in Example 1 and the chemiluminescence in Example 2 of the present application, anti-Nephrin autoantibodies in the samples were detected, and the relative light unit (RLU) values were obtained.

In this example, during preparation of magnetic microparticles, the coating amount of the magnetic beads was 5 µg/mg, the particle size of the carboxyl magnetic beads was 1.5 µm, and the blocking agent was bovine serum albumin.

Further, during detection of anti-Nephrin autoantibodies in samples, the concentration of the magnetic microparticle working solution was 0.2 mg/mL, the concentration of the labeled antibody working solution was 0.04 µg/mL, the amount of the sample was 5 µL, and the first step of the reaction procedure was reacted for about 10 minutes.

Table 1 shows the influence of different types of magnetic beads on the detection of luminescence values for anti-Nephrin autoantibodies in samples according to an example of the present application.

**Table 1 Optimization of Different Types of Magnetic Beads**

| | | Toysl Magnetic Beads | Carboxyl Magnetic Beads |
|---|---|---|---|
| | | RLU | RLU |
| Experimental group | 1 | 34647 | 4439 |
| | 2 | 33933 | 1148 |
| | 3 | 33828 | 1321 |
| | 4 | 34347 | 848 |
| | 5 | 34136 | 964 |
| | 6 | 34857 | 1060 |
| | 7 | 34724 | 2012 |
| | 8 | 35073 | 1115 |
| | 9 | 32277 | 952 |
| | 10 | 34647 | 4439 |
| | Mean | 34202 | 1540 |
| | 1 | 35444 | 1125 |
| | 2 | 33385 | 1662 |
| | 3 | 33935 | 1172 |
| | 4 | 34115 | 975 |
| | 5 | 33853 | 912 |
| Control group | 6 | 34191 | 1059 |
| | 7 | 38938 | 1353 |
| | 8 | 32795 | 910 |
| | 9 | 28620 | 1164 |
| | 10 | 35444 | 1125 |
| | Mean | 35920 | 1148 |
| S/N | | 0.95 | 1.34 |

Where S represents the average value of the detection results (i.e., luminescence values) for samples in the experimental group, N represents the average value of the detection results (i.e., luminescence values) for samples in the control group, and S/N refers to the signal-to-noise ratio. A larger S/N value indicates greater distinction degree between the detection results for positive samples and negative samples when using the kit for detecting the samples, and less prone to generate false results (e.g., false positives or false negatives), thereby indicating better sensitivity and accuracy of the kit. In the following examples, the same signs have similar meanings as in this example and will not be reiterated. From Table 1, it can be seen that the S/N value of carboxyl magnetic beads reaches 1.34, much higher than that of Toysl beads, indicating better signal-to-noise ratio, so that the carboxyl magnetic bead can be the optimized magnetic bead type.

### 2. Influence of Different Coating Amounts of Antigen on Sample Reactivity

In this example, 4 clinical anti-Nephrin autoantibody-positive samples and 4 clinical anti-Nephrin autoantibody-negative samples were selected. Magnetic microparticles with different amounts of Nephrin antigen coated on carboxyl magnetic beads were prepared. Using other reagents from the kit in Example 1 and the chemiluminescence in Example 2 of the present application, anti-Nephrin autoantibodies in the samples were detected, and the relative light unit (RLU) values were obtained.

In this example, the particle size of the carboxyl magnetic beads was 1.5 µm, and the blocking agent was bovine serum albumin.

Further, during detection of anti-Nephrin autoantibodies in the samples, the concentration of the magnetic microparticle working solution was 0.2 mg/mL, the concentration of the labeled antibody working solution was 0.04 µg/mL, the amount of the sample was 5 µL, and the first step of the reaction procedure was reacted for about 10 minutes.

Table 2 shows the influence of different coating amounts of magnetic beads on the detection of luminescence values for anti-Nephrin autoantibodies in samples according to an example of the present application.

**Table 2 Optimization of Different Antigen Coating Amounts of Magnetic Beads**

| | | Coating Amounts of Magnetic Beads | | | |
|---|---|---|---|---|---|
| | | 5 µg/mg | 10 µg/mg | 15 µg/mg | 20 µg/mg |
| Experimental group | 1 | 6356 | 25870 | 42791 | 61877 |
| | 2 | 14199 | 11822 | 10821 | 10515 |
| | 3 | 4671 | 21999 | 37223 | 48483 |
| | 4 | 4709 | 18423 | 5093 | 10055 |
| | Mean | 7484 | 19529 | 23982 | 32733 |
| Control group | 1 | 1979 | 4158 | 5742 | 7546 |
| | 2 | 1970 | 3221 | 4104 | 4967 |
| | 3 | 1080 | 1529 | 2005 | 2886 |
| | 4 | 563 | 819 | 1345 | 2714 |
| | Mean | 1398 | 2432 | 3299 | 4529 |
| S/N | | 5.35 | 7.92 | 7.27 | 7.23 |

From Table 2, it can be seen that the signal-to-noise ratio is best at a coating amount of magnetic beads of 10 µg/mg. Therefore, it is determined that the optimized coating amount of magnetic beads in this example is 10 µg/mg.

### 3. Influence of Different Particle Sizes of Magnetic Beads on Sample Reactivity

In this example, 3 clinical anti-Nephrin autoantibody-positive samples and 3 clinical anti-Nephrin autoantibody-negative samples were selected. Magnetic microparticles with Nephrin antigen-coated carboxyl magnetic beads of different particle sizes were obtained. Using other reagents from the kit in Example 1 and the chemiluminescence in Example 2 of the present application, anti-Nephrin autoantibodies in the samples were detected, and the relative light unit (RLU) values were obtained.

In this example, during preparation of magnetic microparticles, the coating amount of magnetic beads was 10 µg/mg, and the blocking agent was bovine serum albumin.

Further, during detection of anti-Nephrin autoantibodies in the samples, the concentration of the magnetic microparticle working solution was 0.2 mg/mL, the concentration of the labeled antibody working solution was 0.04 µg/mL, the amount of the sample was 5 µL, and the first step of the reaction procedure was reacted for about 10 minutes.

Table 3 shows the influence of magnetic microparticles with magnetic beads of different particle sizes on the detection of luminescence values for anti-Nephrin autoantibodies in samples according to an example of the present application.

**Table 3 Optimization of Magnetic Beads of Different Particle Sizes**

| | | Particle Sizes of Magnetic Beads | | |
|---|---|---|---|---|
| | | 1.0 µm | 1.5 µm | 3.0 µm |
| Experimental group | 1 | 8501 | 5517 | 16983 |
| | 2 | 7243 | 4572 | 13246 |
| | 3 | 30658 | 25790 | 51399 |
| | Mean | 15437 | 11960 | 27209 |
| Control group | 1 | 2852 | 1375 | 1678 |
| | 2 | 2500 | 1854 | 1138 |
| | 3 | 2286 | 1054 | 1372 |
| | Mean | 2546 | 1428 | 1396 |
| S/N | | 12.04 | 8.38 | 19.83 |

From Table 3, it can be seen that the signal-to-noise ratio is best at a particle size of magnetic beads of 3.0 µm. Therefore, it is determined that the optimized particle size of magnetic beads in this example is 3.0 µm.

### 4. Influence of Different Blocking Processes on Sample Reactivity

In this example, 2 clinical anti-Nephrin autoantibody-positive samples and 2 clinical anti-Nephrin autoantibody-negative samples were selected. Carboxyl magnetic microparticles obtained with different blocking processes were prepared. Using other reagents from the kit in Example 1 and the chemiluminescence in Example 2 of the present application, anti-Nephrin autoantibodies in the samples were detected, and the relative light unit (RLU) values were obtained.

In this example, the influence of magnetic microparticles prepared with three blocking agents on the detection results for anti-Nephrin autoantibodies in the samples was tested, wherein:
Blocking agent 1 included 1% (w/v) of bovine serum albumin;
Blocking agent 2 included 0.5% (w/v) of casein; and
Blocking agent 3 included 0.5% (w/v) of fish gelatin.

Further, in this example, during preparation of magnetic microparticles, the coating amount of magnetic beads was 10 µg/mg, the particle size of magnetic beads was 3 µm, and the blocking agent was bovine serum albumin.

Further, during detection of anti-Nephrin autoantibodies in the samples, the concentration of the magnetic microparticle working solution was 0.2 mg/mL, the concentration of the labeled antibody working solution was 0.04 µg/mL, the amount of the sample was 5 µL, and the first step of the reaction procedure was reacted for about 10 minutes.

Table 4 shows the influence of magnetic microparticles prepared with different blocking processes on the detection of luminescence values for anti-Nephrin autoantibodies in samples according to an example of the present application.

**Table 4 Optimization of Different Blocking Processes**

| | | Blocking Processes | | |
|---|---|---|---|---|
| | | Blocking Agent 1 (bovine serum albumin) | Blocking Agent 2 (casein) | Blocking Agent 3 (fish gelatin) |
| Experimental group | 1 | 34102 | 28461 | 32960 |
| | 2 | 29838 | 24236 | 27806 |
| | Mean | 31970 | 26349 | 30383 |
| Control group | 1 | 1602 | 1104 | 984 |
| | 2 | 1032 | 906 | 840 |
| | Mean | 1317 | 1005 | 912 |
| S/N | | 24.27 | 26.22 | 33.31 |

From Table 4, it can be seen that the blocking agent 3 has the best signal-to-noise ratio. Therefore, it is determined that the optimized blocking agent for blocking magnetic beads in this example is fish gelatin.

### Example 4: Optimization of Working Concentrations of Reagents

In this example, the magnetic microparticles used were the optimal magnetic microparticles obtained under the optimization conditions of Example 3. Other reagents were obtained as those in Example 1.

When anti-Nephrin autoantibodies in samples were detected using the kit in Example 1, the mass ratio of magnetic microparticles to labeled antibodies was 250: 1. The concentration of the R1 reagent (i.e., the magnetic microparticle working solution) was set to be 0.1 mg/mL or 0.2 mg/mL, and the concentration of the R2 reagent (i.e., the labeled antibody working solution) was set to be 0.02 µg/mL or 0.04 µg/mL. The optimized dilution concentration was selected based on comprehensive consideration of the signal-to-noise ratio of luminescence values.

Further, during detection of anti-Nephrin autoantibodies in the samples, the amount of the sample was 5 µL, and the first step of the reaction procedure was reacted for about 10 minutes.

Table 5 shows the influence of different concentrations of magnetic microparticles and labeled antibodies on the detection of luminescence values for anti-Nephrin autoantibodies in the samples according to an example of the present application.

**Table 5 Optimization of Different Working Concentrations of Reagents in the Kit**

| Concentrations of R1 Reagent | | 0.1 mg/mL | 0.2 mg/mL |
|---|---|---|---|
| Concentrations of R2 Reagent | | 0.02 µg/mL | 0.04 µg/mL |
| Experimental group | 1 | 14117 | 63533 |
| | 2 | 11366 | 52721 |
| | 3 | 34436 | 26494 |
| | 4 | 3036 | 12621 |
| | Mean | 15739 | 38842 |
| Control group | 1 | 1025 | 2346 |
| | 2 | 828 | 2523 |
| | 3 | 894 | 5639 |
| | 4 | 1046 | 3657 |
| | Mean | 948 | 3541 |
| S/N | | 16.60 | 10.97 |

From Table 5, it can be seen that under the optimized concentration of the R1 reagent (i.e., the magnetic microparticle working solution) of 0.1 mg/mL in combination with the optimized concentration of the R2 reagent (i.e., the labeled antibody working solution) of 0.02 µg/mL, the kit of the present application exhibits the highest signal-to-noise ratio.

### Example 5: Influence of Amounts of Sample on Sample Reactivity

In this example, the magnetic microparticles used were the optimal magnetic microparticles obtained under the optimization conditions of Example 3. Other reagents were obtained as those in Example 1.

When anti-Nephrin autoantibodies in samples were detected using the kit in Example 1, different amounts of the sample were set. The optimized dilution concentration was selected based on comprehensive consideration of the signal-to-noise ratio of luminescence values.

Further, during detection of anti-Nephrin autoantibodies in the samples, the concentration of the magnetic microparticle working solution was 0.1 mg/mL, the concentration of the labeled antibody working solution was 0.02 µg/mL, and the first step of the reaction procedure was reacted for about 10 minutes.

Table 6 shows the influence of different amounts of sample on the detection of luminescence values for anti-Nephrin autoantibodies in samples according to an example of the present application.

**Table 6 Optimization of amounts of sample**

| Amounts of sample (µL) | | 5 | 20 | 50 |
|---|---|---|---|---|
| Experimental group | 1 | 4427 | 14117 | 23908 |
| | 2 | 3539 | 11366 | 19882 |
| | 3 | 1518 | 3036 | 2957 |
| | Mean | 3161 | 9506 | 15582 |
| Control group | 1 | 616 | 1025 | 1249 |
| | 2 | 692 | 828 | 1150 |
| | 3 | 734 | 1046 | 3167 |
| | Mean | 681 | 966 | 1855 |
| S/N | | 4.64 | 9.84 | 8.4 |

From Table 6, it can be seen that the optimized amount of sample after dilution is 20 µL. Under this condition, the kit exhibits the highest signal-to-noise ratio.

### Example 6: Optimization of Reaction Conditions

In this example, the magnetic microparticles used were the optimal magnetic microparticles obtained under the optimization conditions of Example 3. Other reagents were obtained as those in Example 1.

When anti-Nephrin autoantibodies in samples were detected using the kit in Example 1, experiments were carried out by seting different first-step reaction times, while keeping the second-step reaction time unchanged at 10 minutes. The optimized reaction time was selected based on comprehensive consideration of the signal-to-noise ratio of luminescence values.

Further, during detection of anti-Nephrin autoantibodies in samples, the concentration of the magnetic microparticle working solution was 0.1 mg/mL, the concentration of the labeled antibody working solution was 0.02 µg/mL, and the amount of sample was 20 µL.

Table 7 shows the influence of different reaction times on the detection of luminescence values for anti-Nephrin autoantibodies in samples according to an example of the present application.

**Table 7 Optimization of Different Reaction Times**

| Reaction Times (min) | | 10 | 20 |
|---|---|---|---|
| Experimental group | 1 | 12999 | 15659 |
| | 2 | 8168 | 12110 |
| | 3 | 12282 | 14581 |
| | 4 | 6746 | 11096 |
| | Mean | 10049 | 13362 |
| Control group | 1 | 1130 | 1439 |
| | 2 | 897 | 1088 |
| | 3 | 1702 | 2075 |
| | 4 | 1057 | 1225 |
| | Mean | 1197 | 1457 |
| S/N | | 8.40 | 9.17 |

From Table 7, it can be seen that when the second-step reaction time is kept unchanged at 10 minutes, the signal-to-noise ratio shows no significant difference between first-step reaction times of 10 minutes and 20 minutes. Based on comprehensive consideration, a first-step reaction time of 10 minutes is selected, which can shorten the reaction time and improve workflow efficiency of the kit.

### Example 7: Testing of Performance of the Anti-Nephrin Autoantibody Assay Kit

Serum samples from 100 healthy children undergoing physical examinations were detected using the anti-Nephrin autoantibody assay kit, with the maximum signal value of less than 15 AU/mL. Additionally, serum samples from 100 children suffering from nephropathy were detected, with the minimum signal value of greater than 20 AU/mL. Therefore, the cut-off value for the detection kit was set to be 20 AU/mL. If a detection result of a sample is greater than or equal to 20 AU/mL, it is determined as reactivity (i.e., positive); and if a detection result of a sample is less than 20 AU/mL, it is determined as non-reactivity (i.e., negative).

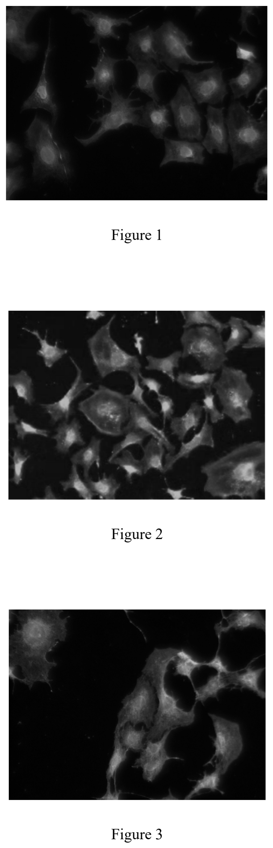

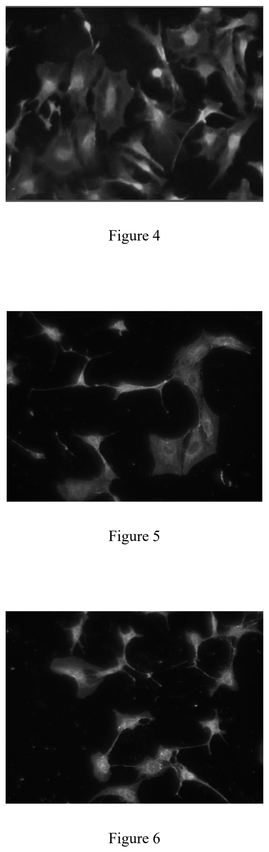

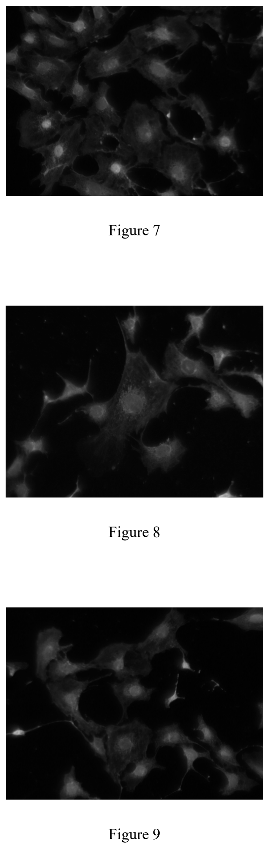

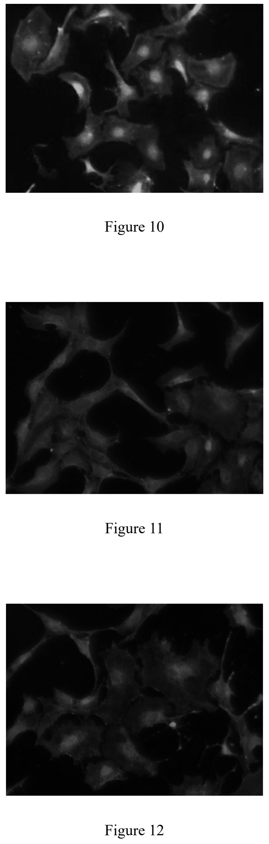

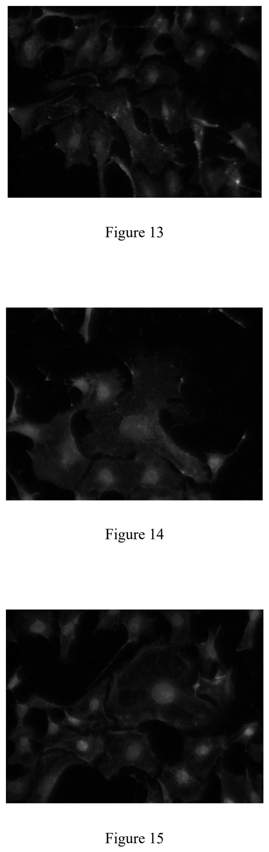

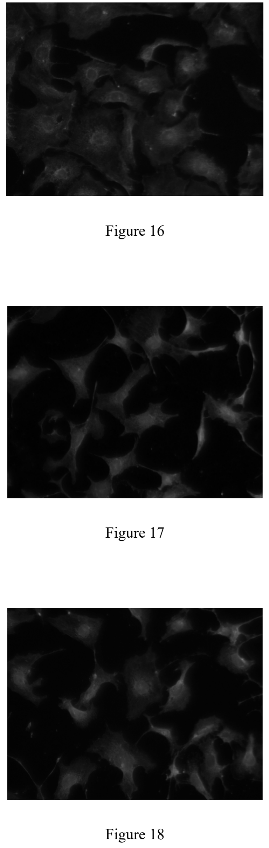

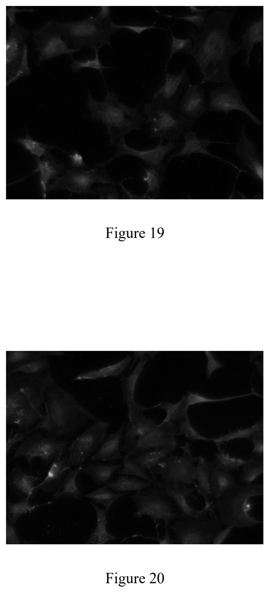

In this example, 20 samples were detected using the indirect immunofluorescence as the gold standard in Example 2, of which 10 samples were detected to be positive (as shown in Figures 1-10), and 10 samples were detected to be negative (as shown in Figures 11-20). The kit prepared in Example 1 and the chemiluminescence in Example 2 were used to detect the content of the anti-Nephrin autoantibodies. The results are shown in Table 8.

**Table 8 Detection Results for Anti-Nephrin Autoantibodies**

| Sample No. | Indirect immunofluorescence | Chemiluminescence |
|---|---|---|
| | | AU/mL |
| 1 | | 11.2 |
| 2 | | 39.81 |
| 3 | | 22.93 |
| 4 | | 20.48 |
| 5 | Positive | 31.72 |
| 6 | | 25.59 |
| 7 | | 26.96 |
| 8 | | 21.46 |
| 9 | | 22.61 |
| 10 | | 36.27 |
| 11 | | 13.02 |
| 12 | | 11.07 |
| 13 | | 20.58 |
| 14 | | 8.59 |
| 15 | Negative | 8.53 |
| 16 | | 8.16 |
| 17 | | 10.64 |
| 18 | | 9.95 |
| 19 | | 7.48 |
| 20 | | 14.60 |
| Cutoff | - | 20.00 |

As shown in Table 8, the positive accuracy rate and the negative accuracy rate detected by the chemiluminescence using the kit of the present application were both 80%.

Further, Figure 21 shows a calibration curve of the anti-Nephrin autoantibody assay kit according to an example of the present application. A serum sample with high luminescence intensity of anti-Nephrin autoantibody was selected as the calibrator and subjected to serial dilution (where RLU values were 2000, 4000, 6000, 8000, 10000, respectively), and corresponding signal values of the calibrator were obtained. A scatter plot of standard RLU as the x-axis versus signal values as the y-axis was drawn. Based on the scatter plot, a linear trendline according to the least squares method was fitted, and the linear equation and coefficient of determination (R²) were plotted, where R² = 1, indicating a good linear relationship. The concentrations for the calibrator include 0.42 AU/mL for CAL1, and 39.3 AU/mL for CAL2. The luminescence intensity value of a sample was matched with the calibration curve of luminescence intensity values of the calibrator to obtain the content in AU of the anti-Nephrin autoantibodies in the sample to be tested.

Figure 22 shows an ROC curve of the detection results of the anti-Nephrin autoantibody assay kit for samples in this example. Based on the ROC curve, it can be seen that the AUC is 0.997.

### Example 8: Sensitivity and Specificity of the Anti-Nephrin Autoantibody Assay Kit

In this example, the inventors tested over 1000 clinically confirmed positive and negative samples and obtained detection results. A cut-off value of ≥20 AU/mL was set as positive, and a cut-off value of <20 AU/mL was set as negative. In this example, the detection accuracy rate for over 1000 clinically positive and negative samples could reach 99% or above. This example further lists detection results for 50 positive samples and 50 negative samples, as shown in Table 9.

**Table 9 Detection Results for Anti-Nephrin Autoantibodies**

| Positive Samples | | | | Negative Samples | | | |
|---|---|---|---|---|---|---|---|
| Sample | AU/mL | Sample | AU/mL | Sample | AU/mL | Sample | AU/mL |
| 1 | 20.58 | 26 | 25.37 | 1 | 13.02 | 26 | 6.78 |
| 2 | 39.81 | 27 | 31.56 | 2 | 11.07 | 27 | 5.05 |
| 3 | 22.93 | 28 | 40.24 | 3 | 11.2 | 28 | 5.18 |
| 4 | 20.48 | 29 | 27.39 | 4 | 8.59 | 29 | 4.86 |
| 5 | 31.72 | 30 | 40.83 | 5 | 8.53 | 30 | 5.38 |
| 6 | 25.59 | 31 | 28.56 | 6 | 8.16 | 31 | 4.68 |
| 7 | 26.96 | 32 | 33.36 | 7 | 10.64 | 32 | 5.59 |
| 8 | 21.46 | 33 | 24.81 | 8 | 9.95 | 33 | 8.10 |
| 9 | 22.61 | 34 | 49.75 | 9 | 7.48 | 34 | 7.66 |
| 10 | 36.27 | 35 | 37.10 | 10 | 14.60 | 35 | 3.81 |
| 11 | 36.55 | 36 | 21.49 | 11 | 4.60 | 36 | 8.78 |
| 12 | 25.59 | 37 | 28.44 | 12 | 4.32 | 37 | 3.72 |
| 13 | 26.96 | 38 | 23.49 | 13 | 4.82 | 38 | 12.50 |
| 14 | 27.15 | 39 | 49.63 | 14 | 4.10 | 39 | 10.54 |
| 15 | 28.50 | 40 | 20.86 | 15 | 6.43 | 40 | 5.13 |
| 16 | 21.46 | 41 | 22.69 | 16 | 5.29 | 41 | 5.46 |
| 17 | 24.88 | 42 | 36.27 | 17 | 8.16 | 42 | 3.32 |
| 18 | 22.90 | 43 | 27.91 | 18 | 5.26 | 43 | 6.12 |
| 19 | 29.33 | 44 | 27.64 | 19 | 4.15 | 44 | 11.95 |
| 20 | 20.09 | 45 | 25.56 | 20 | 7.31 | 45 | 13.30 |
| 21 | 33.38 | 46 | 26.36 | 21 | 3.61 | 46 | 6.81 |
| 22 | 26.88 | 47 | 31.93 | 22 | 9.30 | 47 | 10.80 |
| 23 | 22.61 | 48 | 30.99 | 23 | 4.09 | 48 | 4.08 |
| 24 | 23.68 | 49 | 42.91 | 24 | 2.94 | 49 | 8.01 |
| 25 | 24.69 | 50 | 33.18 | 25 | 4.46 | 50 | 5.27 |

In this example, the detection rates for negative samples and positive samples are both close to 100%. The applicant further calculated: among positive samples, the number of the positive samples that were still detected as positive by the kit of the present application was counted as TP, and the number of the positive samples that were detected as negative was counted as FN; among negative samples, the number of the negative samples that were detected as positive by the kit of the present application was counted as FP, and the number of the negative samples that were still detected as negative was counted as TN.

The sensitivity of the kit is detemined by a formula: Sensitivity = TP / (TP + FN) * 100%.

The specificity of the kit is detemined by a formula: Specificity = TN / (TN + FP) * 100%.

Based on statistics and calculation, the sensitivity and specificity of this kit are both close to 100%.

The above embodiments are only for illustrating the present application and are not intended to limit the present application. Those skilled in the art can make various changes and modifications without departing from the scope of the present application. Therefore, all equivalent technical solutions should also fall within the disclosed scope of the present application.

## Claims

1. A magnetic microparticle specifically binding to an anti-Nephrin autoantibody in a sample, comprising a Nephrin antigen and a magnetic bead, the Nephrin antigen being coated on a surface of the magnetic bead; wherein the magnetic bead is a carboxyl magnetic bead, and a particle size of the carboxyl magnetic bead is about 1 µm to 5 µm;
optionally, the particle size of the carboxyl magnetic bead is about 1 µm to 3 µm;
optionally, a particle size of the magnetic bead is about 3 µm.

2. The magnetic microparticle according to claim 1, wherein the Nephrin antigen is a polypeptide or a fragment thereof capable of specifically binding to an anti-Nephrin autoantibody in a mammal, and the Nephrin antigen has a sequence as set forth in SEQ ID NO: 1.

3. A preparation method for the magnetic microparticle according to claim 1 or 2, comprising:
obtaining a mixed solution comprising an activated magnetic bead,
adding a Nephrin antigen to the mixed solution and mixing uniformly, the Nephrin antigen being a polypeptide or a fragment thereof capable of specifically binding to an anti-Nephrin autoantibody in a mammal;
suspending and reacting the mixed solution for about 1 to 2 hours at a temperature of about 20 to 25 °C;
washing a reacted product with a blocking agent, wherein the blocking agent comprises about 1% (w/v) of bovine serum albumin, about 0.5% (w/v) of casein, or about 0.5% (w/v) of fish gelatin; and
obtaining a Nephrin antigen-coated magnetic bead.

4. The preparation method according to claim 3, further comprising diluting the Nephrin antigen-coated magnetic bead in a diluent to obtain a magnetic microparticle-diluted solution;
wherein a concentration of the magnetic microparticles in the magnetic microparticle-diluted solution is about 0.1 to 0.2 mg/mL.

5. The preparation method according to any one of claims 3 to 4, wherein the diluent comprises about 0.80 to 1.30 g/L of tris(hydroxymethyl)aminomethane, about 5.00 to 8.00 g/L of tris(hydroxymethyl)aminomethane hydrochloride, about 7.00 to 11.00 g/L of sodium chloride, about 1.00 to 5.00 g/L of Triton-X 100, about 0.20 to 0.70 g/L of Tween-20, and about 0.20 to 0.70 g/L of ProClin 300;
optionally, the diluent comprises about 1.12 g/L of tris(hydroxymethyl)aminomethane, about 6.42 g/L of tris(hydroxymethyl)aminomethane hydrochloride, about 9.00 g/L of sodium chloride, about 3.00 g/L of Triton-X 100, about 0.50 g/L of Tween-20, and about 0.50 g/L of ProClin 300.

6. The preparation method according to claim 3, wherein a ratio of the magnetic bead added to the Nephrin antigen is: adding about 5 to 20 µg of the Nephrin antigen, optionally, adding about 10 to 20 µg of the Nephrin antigen, to a magnetic bead diluent containing about 1 mg of the magnetic bead.

7. An anti-Nephrin autoantibody assay kit, comprising
the magnetic microparticle according to claim 1 or 2, or the magnetic microparticle prepared by the preparation method according to any one of claims 3 to 6.

8. The kit according to claim 7, further comprising a chemiluminescent label-labeled IgG antibody and a diluent thereof; optionally, a concentration of the chemiluminescent label-labeled IgG antibody in the diluent thereof is 0.02 to 0.04 µg/mL.

9. The kit according to any one of claims 7 to 8, wherein the chemiluminescent label is selected from acridinium ester, acridinium sulfonamide, acridinium toluenesulfonamide, acridinium p-methylsulfonamide, or acridinium trifluoromethanesulfonamide;
optionally, the chemiluminescent label is acridinium ester.

10. The kit according to any one of claims 7 to 9, wherein the chemiluminescent label-labeled IgG antibody is diluted in a label diluent, and
the label diluent comprises about 0.30 to 0.70 g/L of sodium dihydrogen phosphate, about 15.00 to 18.00 g/L of disodium hydrogen phosphate, about 35.00 to 45.00 g/L of sodium chloride, about 7.00 to 13.00 g/L of bovine serum albumin, about 3.00 to 7.00 g/L of Triton-X 405, and about 0.30 to 0.70 g/L of ProClin 300;
optionally, the label diluent comprises 0.50 g/L of sodium dihydrogen phosphate, 16.75 g/L of disodium hydrogen phosphate, about 40.00 g/L of sodium chloride, about 10.00 g/L of bovine serum albumin, about 5.00 g/L of Triton-X 405, and about 0.50 g/L of ProClin 300.

11. The kit according to any one of claims 7 to 10, further comprising a sample diluent;
the sample diluent comprises about 0.50 to 1.50 g/L of tris(hydroxymethyl)aminomethane, about 5.00 to 8.00 g/L of tris(hydroxymethyl)aminomethane hydrochloride, about 7.00 to 11.00 g/L of sodium chloride, and about 17.00 to 23.00 g/L of bovine serum albumin;
optionally, the sample diluent comprises about 1.00 g/L of tris(hydroxymethyl)aminomethane, about 6.58 g/L of tris(hydroxymethyl)aminomethane hydrochloride, about 9.00 g/L of sodium chloride, and about 20.00 g/L of bovine serum albumin;
optionally, the sample diluent further comprises about 0.30 to 0.70 g/L of Tween 20, and about 0.30 to 0.70 g/L of ProClin 300;
optionally, the sample diluent further comprises about 0.50 g/L of Tween 20 and about 0.50 g/L of ProClin 300.

12. The kit according to any one of claims 7 to 11, further comprising a calibrator and a calibrator buffer solution, wherein the calibrator comprises the anti-Nephrin autoantibody.

13. The kit according to any one of claims 7 to 12, wherein the calibrator buffer solution comprises about 0.10 to 0.30 g/L of tris(hydroxymethyl)aminomethane, about 1.00 to 1.60 g/L of tris(hydroxymethyl)aminomethane hydrochloride, about 7.00 to 11.00 g/L of sodium chloride, about 3.00 to 7.00 g/L of bovine serum albumin, about 0.30 to 0.70 g/L of Tween, and about 0.30 to 0.70 g/L of ProClin 300;
optionally, the calibrator buffer solution comprises about 0.20 g/L of tris(hydroxymethyl)aminomethane, about 1.32 g/L of tris(hydroxymethyl)aminomethane hydrochloride, about 9.00 g/L of sodium chloride, about 5.00 g/L of bovine serum albumin, about 0.50 g/L of Tween, and about 0.50 g/L of ProClin 300.

14. A method for detecting an anti-Nephrin autoantibody in a sample using the magnetic microparticle according to claim 1 or 2, the magnetic microparticle prepared by the preparation method according to any one of claims 3 to 6, or the kit according to any one of claims 7 to 13, comprising:
adding about 20 to 50 µL of a sample to be tested to a sample diluent and mixing uniformly;
adding a Nephrin antigen-coated magnetic bead or a magnetic microparticle-diluted solution, and reacting for 10 to 20 minutes;
adding an acridinium ester-labeled mouse anti-human IgG antibody or an acridinium ester-labeled mouse anti-human IgG antibody diluted in a label diluent, and reacting for 10 minutes;
adding a pre-excitation solution and an excitation solution; and
detecting a relative luminescence intensity,
wherein a volume ratio of the sample to be tested, the magnetic microparticle-diluted solution, the acridinium ester-labeled mouse anti-human IgG antibody diluted in the label diluent, and the sample diluent that are added is (15 to 25): (40 to 60): (80 to 120): (80 to 120), preferably 20: 50: 100: 100.

15. The method according to claim 14, wherein the pre-excitation solution comprises 0.80 to 1.70 % (w/v) of hydrogen peroxide, and the excitation solution comprises about 0.2 to 0.5 mol/L of sodium hydroxide; optionally, the pre-excitation solution comprises about 1.32% (w/v) of hydrogen peroxide, and the excitation solution comprises about 0.35 mol/L of sodium hydroxide.

16. The method according to any one of claims 14 to 15, further comprising:
determining a calibration curve based on concentrations of a calibrator and luminescence intensity values thereof;
matching the relative luminescence intensity of the sample with the calibration curve of luminescence intensity values of the calibrator; and
obtaining a content of the anti-Nephrin autoantibody in the sample to be tested.

17. The method according to any one of claims 14 to 16, wherein the sample is serum.
